# EUROPÄISCHE PATENTSCHRIFT

(11) **EP 0 752 709 B1**
(45) Veröffentlichungstag und Bekanntmachung des Hinweises auf die Patenterteilung: **24.03.1999**
(21) Anmeldenummer: 96110720.8
(22) Anmeldetag: 03.07.1996
(51) Int. Cl.: G21G 1/06, A61K 51/10

(54) **Verfahren zur Erzeugung von Actinium-225**
Process for producing actinium-225
Procédé pour produire l'actinium-225

(30) Priorität: 03.07.1995 LU 88636
(43) Veröffentlichungstag der Anmeldung: 08.01.1997
(73) Patentinhaber: EUROPÄISCHE ATOMGEMEINSCHAFT (EURATOM), 2920 Luxembourg (LU)
(72) Erfinder: Koch, Lothar, 76356 Weingarten (DE); Fuger, Jean, 75045 Wössingen (DE); van Geel, Jacques, 76275 Ettlingen-Oberweier (DE)
(74) Vertreter: Weinmiller, Jürgen

(56) Entgegenhaltungen:
- EP-A- 0 443 479
- WO-A-90/15625
- CHEMICAL ABSTRACTS, Bd. 115, Nr. 12, 23 September 1991, Columbus, Ohio, US; Zusammenfassung Nr. 11559100, BEYER ET AL 'PREPARATION AND CHEMICAL SEPARATION OF ACTINIUM-225.' Seite 641; Spalte L; XP002009313 & ZENTRALINST. KERNFORSCH. , ROSSENDORF, Bd. ZfK, Nr. 711, DRESDEN, Seiten 17-20
- DATABASE INIS INTERNATIONAL ATOMIC ENERGY AGENCY (IAEA), VIENNA, AT AN: 21(17):66714, BEYER ET AL: "COMPARISON OF THE BIODISTRIBUTION OF 225AC AND RADIOLANTHANIDES AS CITRATES COMPLEXES" XP002009314 & ISOTOPENPRAXIS, Bd. 26, Nr. 3, DDR, Seiten 111-114

## Beschreibung

Die Erfindung bezieht sich auf ein Verfahren zur Erzeugung von Actinium-225 aus Radium-226 mit Hilfe einer von einer Kernstrahlung ausgelösten Kernreaktion.

Ein solches Verfahren ist z.B. aus der EP-0 443 479-B1 bekannt. Gemäß diesem Verfahren wird das Radium-226 in einem thermischen Neutronenfluß eines Kernreaktors bestrahlt, wobei u.a. Thorium-229 entsteht. Der Thoriumanteil wird dann chemisch isoliert und daraus wird das entstehende Radium-225 abgetrennt. Schließlich wird aus dem Radium das einwachsende Actinium-225 isoliert.

Radiotherapeutische Methoden zur lokalen Bekämpfung von Krebserkrankungen (Metastasen) gewinnen mit Fortschritten in der Molekularbiologie immer mehr an Bedeutung. Im Prinzip werden dabei kurzlebige alphastrahlende Nuklide in monoklonale Antikörper eingebaut, die, in den Körper des Patienten gebracht, von den malignen Zellen bevorzugt aufgenommen werden und diese durch intensive Bestrahlung mit sehr kurzer Reichweite zerstören. An das Radionuklid werden dabei besondere Anforderungen gestellt: Es muß sich für die Konjugation an einen geeigneten Antikörper binden lassen, muß eine kurze Halbwertzeit haben (in der Größenordnung von einigen Stunden) und seine Zerfallsprodukte müssen chemisch und radiologisch weitgehend unschädlich sein.

Unter den möglichen Kandidaten für ein derartiges Radionuklid spielen Actinium-225 und Wismut eine hervorragende Rolle, und zwar bezüglich Wismut entweder das Isotop Bi-212 (Halbwertzeit 60,6 Minuten) oder das Isotop Bi-213 (Halbwertzeit 47 Minuten). Die Herstellung von Bi-212 für medizinische Zwecke wurde in der Zeitschrift Ing. J. Nucl. Med. Biol. 9 (1982), Seite 83 beschrieben. Dieses Isotop hat allerdings den Nachteil, daß als Folgeprodukt ein gamma-aktives Thalliumisotop auftritt, das zu einer unerwünschten Strahlenbelastung des Patienten führt.

Dies gilt zwar nicht für Bi-213, jedoch ist dieses Isotop nicht in ausreichenden Mengen verfügbar und kann auch bisher nicht mit vertretbarem Aufwand in diesen Mengen aus U-233 hergestellt werden. Dieses Uranisotop U-233 bildet über mehrere Zerfallsschritte Actinium-225 und dieses schließlich Bi-213.

Der Nachteil dieses bekannten Verfahrens liegt in der unerwünscht großen Verunreinigung des Actinium-225 mit anderen Actinium-Isotopen oder anderen Folgeprodukten der Zerfallsreihen von Thorium-232, Uran-238 und Uran-235.

Aufgabe der Erfindung ist es, Ac-225 höherer Reinheit herzustellen und diese Produktion besser an den Bedarf anzupassen, als dies nach dem bekannten Verfahren der Fall ist. Dort führt nämlich die Bestrahlung von Ra-226 im Reaktor zu Thorium-229, das wegen seiner langen Halbwertszeit von 7340 Jahren in großen Mengen hergestellt werden muß, um vernünftige Aktivitätsmengen des Nuklides Ac-225 zu erhalten.

Diese Aufgabe wird durch das im Patentanspruch definierte Verfahren gelöst.

Nachfolgend wird die Erfindung anhand eines Ausführungsbeispiels und der beiliegenden Zeichnungen näher erläutert.

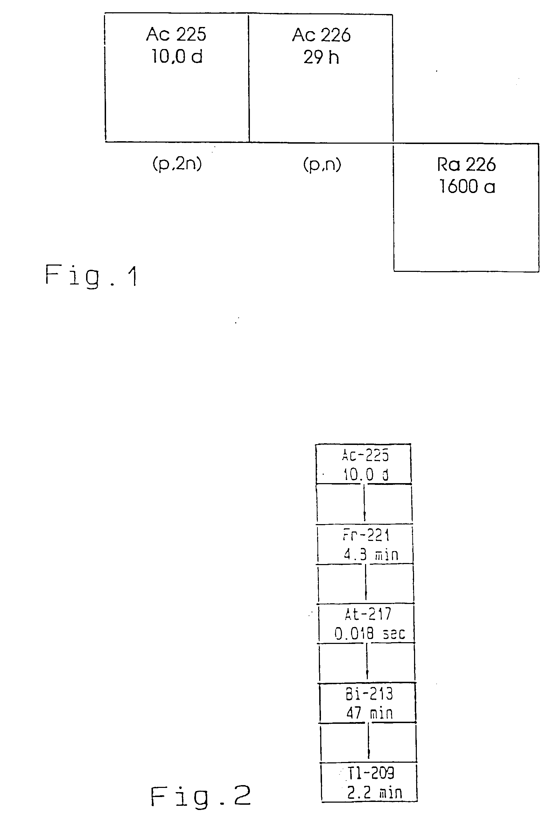

Figur 1 zeigt einen Ausschnitt aus der Nuklidkarte mit den für das erfindungsgemäße Verfahren wichtigen Isotopen der Elemente Ra und Ac.

Figur 2 zeigt die natürliche Zerfallskette von Ac-225 zu dem therapeutisch wichtigen Bi-213.

Ausgangsprodukt des Verfahrens ist Radium-226. Man erzeugt ein Target aus diesem Material und setzt es in ein Elektronenzyklotron ein. Dort wird es mit beschleunigten Protonen beschossen, so daß zwei Kernreaktionen erfolgen: Bei der hier wichtigeren wandelt sich der metastabile Radiumkern unter Aussendung von zwei Neutronen direkt in Actinium-225 um. Gleichzeitig entsteht aber durch Emission nur eines Neutrons auch Actinium-226, wenn auch mit geringerer Ausbeute. Da letzteres ebenfalls eine Zerfallskette besitzt, die in Wismut-210 bzw. Wismut-214 zerfällt, kann durch Wahl der Aufarbeitungszeit des bestrahlten Radium-Targets eine Trennung der Proben dergestalt erfolgen, daß aufgrund der geringeren Halbwertszeit des Actinium-229 mit 29 h so lange mit der Aufarbeitung gewartet wird, bis dies auf einen akzeptablen Untergrund zerfallen ist. Das dann verbleibende Actinium-225 mit einer Halbwertszeit von 10 d liegt dann immer noch in ausreichender Konzentration vor, um es aus dem Nuklidgemisch abgetrennt für die therapeutische Anwendung mit seinen Tochternukliden zu separieren.

Der Vorteil gegenüber dem im Patent 0 443 479 B1 erwähnten Verfahren liegt darin, daß man die Produktion dieser Nuklide elastisch an den Bedarf anpassen kann, da die Reaktion mit Protonen mittels eines Zyklotrons leicht Mengen dieser Nuklide im Curie-Bereich erzeugt. Im Gegensatz dazu führt die Bestrahlung von Radium-226 durch Reaktorneutronen zu Thorium-229, das in größeren Mengen hergestellt werden muß (aufgrund seiner größeren Halbwertszeit von 7340 a), um vergleichbare Aktivitätsmengen des Nuklides Actinium-225 zu erhalten. Darüber hinaus führt die Produktion von Thorium-229 immer zu erheblichen Beimengungen von Thorium-228, das die Handhabung und Aufarbeitung des Thoriums und die Gewinnung des Actinium-225 erheblich erschwert.

## Patentansprüche

1. Verfahren zur Erzeugung von Actinium-225 aus Radium-226 mit Hilfe einer von einer Kernstrahlung ausgelösten Kernreaktion, dadurch gekennzeichnet, daß auf ein Target aus Radium-226 in einem Zyklotron beschleunigte Protonen geschossen werden, so daß sich metastabile Kerne des Radium unter Aussendung von zwei Neutronen in Actinium-225 umwandeln, daß dann nach einer Wartezeit, in der das gleichzeitig aufgrund der Aussendung nur eines Neutrons gebildete Actinium-226 wegen seiner deutlich kürzeren Halbwertszeit größtenteils zerfällt, das Actinium chemisch abgetrennt wird, bei dem es sich nun fast ausschließlich um das Isotop Ac-225 handelt.

## Claims

1. A method for producing actinium-225 from radium-226 by means of a nuclear reaction initiated by a nuclear irradiation, characterized in that protons accelerated in a cyclotron are projected onto a target of radium-226, so that metastable radium nuclei are transformed into actinium-225 while emitting two neutrons, and then, after a waiting period, during which the actinium-226, which has been created simultaneously due to the emission of only one neutron, decays mostly due to its considerably shorter half-lifetime, actinium is chemically separated which now consists almost exclusively of the isotope Ac-225.

## Revendications

1. Procédé pour la production d'actinium 225 à partir de radium 226 à l'aide d'une réaction nucléaire causée par un rayonnement nucléaire, caractérisé en ce que des protons accélérés dans un cyclotron sont projetés sur une cible en radium 226, ce qui fait que des noyaux métastables du radium se transforment en actinium 225 en émettant deux neutrons, et qu'après une période d'attente, pendant laquelle l'actinium 226 formé simultanément à cause de l'émission d'un seul neutron se désintègre dû à sa demi-vie radioactive notablement plus courte, l'actinium est isolé chimiquement, qui est alors presque exclusivement l'isotope actinium 225.
